(19) Europäisches Patentamt

European Patent Office

Office européen des brevets

(11) Veröffentlichungsnummer: **0 292 809 B1**

(12) **EUROPÄISCHE PATENTSCHRIFT**

(45) Veröffentlichungstag der Patentschrift: **20.07.94**

(21) Anmeldenummer: **88107703.6**

(22) Anmeldetag: **13.05.88**

(51) Int. Cl.⁵: **G01N 33/543**, //G01N33/574, G01N33/68

Die Akte enthält technische Angaben, die nach dem Eingang der Anmeldung eingereicht wurden und die nicht in dieser Patentschrift enthalten sind.

(54) **Verfahren zur Bestimmung von Proteinen und Mittel dazu.**

(30) Priorität: **23.05.87 DE 3717402**

(43) Veröffentlichungstag der Anmeldung:
**30.11.88 Patentblatt 88/48**

(45) Bekanntmachung des Hinweises auf die Patenterteilung:
**20.07.94 Patentblatt 94/29**

(84) Benannte Vertragsstaaten:
**AT BE CH DE FR GB IT LI LU NL SE**

(56) Entgegenhaltungen:
**EP-A- 0 008 338      EP-A- 0 132 948
EP-A- 0 133 272      EP-A- 0 171 946
DE-A- 3 500 190      US-A- 4 357 310**

(73) Patentinhaber: **BEHRINGWERKE Aktiengesellschaft
Postfach 1140
D-35001 Marburg(DE)**

(72) Erfinder: **Kapmeyer, Wolfgang, Dr.
Reinhardswaldstrasse 5
D-3550 Marburg(DE)**
Erfinder: **Schmidtberger, Rudolf
Salegrund 1
D-3550 Marburg(DE)**

## Beschreibung

Die Erfindung betrifft ein Verfahren zur quantitativen Bestimmung von Proteinen in Korperflüssigkeiten mit Hilfe einer Festphasen-Immunreaktion mit kovalent an die Festphase gebundenem Partner der Immunreaktion in Gegenwart eines Tensids sowie die Verwendung eines Tensids zu diesem Zweck.

Zur quantitativen Bestimmung von Immunglobulinen und anderen Serumproteinen sind zahlreiche nephelometrische und turbidimetrische Methoden bekannt. Bei diesen Verfahren liegt die untere Nachweisgrenze bei etwa 5 $\mu$g/ml. Für viele diagnostische Fragestellungen reicht diese Empfindlichkeit nicht aus.

Es ist bekannt, die Empfindlichkeit immunologischer Bestimmungsverfahren durch die Verwendung von Indikator-oder Trägerteilchen zu erhöhen, die mit einem der immunchemischen Reaktanten des betreffenden Verfahren beladen sind. Als Trägermaterial können zum Beispiel rote Blutkörperchen, Zellen einer Zellkultur oder partikuläre Polymere, besonders Latexpartikel mit einem Durchmesser von 0,02 bis 5 $\mu$m verwendet werden.

Ein solcher "partikelverstärkter" nephelometrischer oder turbidimetrischer Test kann Proteine bis zu Konzentrationen von etwa 50 ng/ml sicher bestimmen.

Zur Bestimmung eines Antigens kommt ein festphasen-gebundener Antikörper zum Einsatz, zur Bestimmung eines Antikörpers ein festphasen-gebundenes Antigen. In beiden Fällen agglutinieren die Polymerpartikel durch die Immunreaktion. Daraus resultiert eine Größenzunahme der Agglutinate und das Streulichtsignal oder die Trübung des Reaktionsansatzes nehmen zu.

Bei einem partikelverstärkten nephelometrischen oder turbidimetrischen Test oder einem Enzymimmunoassay finden Immunreaktionen an Festphasen statt. Es ist eine Eigen schaft dieser Festphasen, unspezifisch Proteine aus der Probe zu adsorbieren. Bei der Messung von Analyten aus Serum oder anderen Körperflüssigkeiten können zum Beispiel Albumin, Globuline oder Lipoproteine zu einer Bedeckung der Festphase führen. Hierbei kann es zu einem Abdecken der immunreaktiven Komponenten auf der Festphase kommen.

Daraus resultieren falsche zu niedrige und schwankende Ergebnisse in Bestimmungen von Spurenproteinen in Körperflüssigkeiten. Es werden jedoch auch falsche zu hohe Werte gemessen. Beide Fälle, falsche zu niedrige und falsche zu hohe Ergebnisse treten bei Messungen mit partikelverstärkten Reagenzien nach dem Stand der Technik auf und verhindern somit eine sichere und reproduzierbare Messung.

Aus EP-A-0 133 272 (USP 4,448,908) ist ein Verfahren bekannt, worin dem Inkubationsmilieu einer Immunreaktion zwischen einem löslichen und einem an eine Festphase adsorbierten Partner ein Detergenz vorzugsweise in einer Konzentration bis zu 0,01 % zugegeben wird.

In EP-A-0 132 848 ist die Verwendung von Detergenzien in heterogenen Immunoassays beschrieben.

In EP-A-0 008 338 ist die Verwendung von Detergenzien zur Trübungsaufhellung in immunchemischen Verfahren zur Bestimmung von Serumproteinen durch Trübungsmessung beschrieben.

Überraschenderweise wurde gefunden, daß von der "Serummatrix" herrührende Störungen bei Festphasen-Immunreaktionen wie partikelverstärkten nephelometrischen oder turbidimetrischen Testen, bei denen der an der Festphase gebundene Partner der Immunreaktion kovalent gebunden ist, dadurch vermieden werden können, daß man die Messung in Gegenwart einer Verbindung der Formel I durchführt.

$$CH_3-(CH_2)_m-R-(CH_2-CH_2-O)_n-H \qquad I$$

worin

R = NH oder CH=CH-$(CH_2)_p$-O mit m = 3 - 26,

n = 7 - 40 und

p = 5 - 15

Gegenstand der Erfindung ist daher ein Verfahren zur Bestimmung eines Partners einer immunchemischen Reaktion, in welchem einer der Partner, in einer wässrigen Flüssigkeit gelöst, mit dem anderen Partner, kovalent an eine Festphase gebunden, reagieren gelassen wird, dadurch gekennzeichnet, daß die Reaktion in Anwesenheit einer Verbindung der Formel I mit den angegebenen Definitionen ablaufen gelassen wird.

Bevorzugt ist eine Verbindung der Formel I mit

R = CH=CH-$(CH_2)_p$-O und

m = 3-26, n = 7-40 und p = 5-15,

besonders bevorzugt

m = 15-17, n = 25 und p = 6-8,

ganz besonders bevorzugt

m = 6-8, n = 25 und p = 6-8.

Besonders bevorzugt ist auch eine Mischung von Verbindungen der Formel I, die 20-35% Palmitylalkohol veräthert mit Polyethylenglycol des Polymerisationsgrads P = 25, 15-30% Stearylalkohol veräthert mit Polyethylenglycol (P = 25) und 30-60% Oleylalkohol veräthert mit Polyethylenglycol (P = 25) enthält und mit der Bezeichnung [R]Genapol T 250 von der Firma Hoechst AG, Frankfurt, FRG vertrieben wird.

Die Verbindung oder Verbindungen der Formel I werden in einer Menge zugesetzt, daß eine Konzentration von mehr als 0,01 bis zu 3 g/100 g vorzugsweise 0,1 bis 1 g/100 g Flüssigkeit erreicht wird.

Das Reaktionsgemisch kann weiterhin eine Aminosäure, vorzugsweise Glycin, vorzugsweise in einer Konzentration von 0,05-0,2 mol/l, ein Neutralsalz vorzugsweise Kochsalz in einer Konzentration von 0,05-0,5 mol/l, Polyethylenglycol vorzugsweise in einer Konzentration von 4 g/100 g und/oder Eikosa-oxyethylen-sorbitan-laurinsäureester ([R]Tween 20) vorzugsweise in einer Konzentration von 0,2-1 g/100 g enthalten.

Die Verbindung der Formel I wird vorzugsweise dem 0,1 mol/l Glycinpuffer, pH 8,0 zugesetzt. Es wird vorzugsweise etwa 30 Minuten bei Raumtemperatur mit dem zu testenden Serum inkubiert und vorzugsweise in einem Nephelometer gemessen.

Die Verwendung kovalent gebundener Reaktionspartner stellt wegen möglicher unspezifischer Ablösung von adsorbierten Reaktionspartnern einen Vorteil dar. Die Verwendung einer Detergenz-Konzentration von bis zu 3 g/100 g bedeutet eine sehr viel größere Wirksamkeit des Detergenzes gegenüber Störeffekten und auf diese Weise können nephelometrische oder turbidimetrische partikelverstärkte Teste unter Eliminierung von Störfaktoren durchgeführt werden.

Als Festphase kommen vor allem Latexpartikel in Frage, die als Dispersion vorliegen.

Zur kovalenten Bindung eines Antikörpers oder Antigens an solche Partikel können beispielsweise Latices gebraucht werden, die Carboxygruppen tragen, die in aktivierte Ester umgewandelt oder mit Carbodiimiden aktiviert und mit dem Antikörper oder dem Antigen reagieren gelassen werden können. Es können aber auch epoxi-, aldehyd- oder aromatische aminogruppen-tragende Latices verwendet werden, wobei im Falle von Aldehydgruppen die kovalente Bindung durch reduktive Aminierung, im Falle aromatischer Aminogruppen durch Diazotierung bewirkt werden kann.

Bevorzugt wird ein festphasen-gebundener Antikörper oder ein solches Antigen durch Reaktion eines Antikörpers oder Antigens mit einem acetalgruppen-tragenden Latex, wie er in EP-A-0 080 614 beschrieben ist, hergestellt.

Als bevorzugt an die Festphase gebundene Antikörper werden solche gegen alpha-Fetoprotein (AFP), Ferritin, Carcino Embryonales Antigen (CEA), Myoglobin, Beta-2-Mikroglobulin oder Immunglobulin E verwendet.

Wird ein auf diese Weise erhaltender AFP-Test ohne eine Verbindung der Formel I durchgeführt, so erhält man für 20 verschiedene Seren, die alle mit 200 ng/ml AFP versetzt wurden, sehr unterschiedliche Werte (Tabelle 1). Es werden im Mittel nur 90,8% des eingesetzten AFP gefunden. Der Variationskoeffizient ist mit 17,5% sehr hoch.

Wird dagegen der AFP-Test unter Zusatz von 1 g/100 ml [R]Genapol durchgeführt, so findet man für 20 verschiedene Seren, jeweils mit 200 ng/ml AFP versetzt, Serum-unabhängig immer den richtigen Wert (Tabelle 1), das heißt man findet die eindosierte Menge AFP wieder.

Tabelle 1

Meßwerte im nephelometrischen Test (zugegebene
AFP-Konzentration: 200 ng/ml)

| Serum-Nr. | Gefundene AFP-Konzentration (ng/ml) | |
|---|---|---|
| | Stand der Technik | Erfindungsgemäß |
| 1 | 153 | 188 |
| 2 | 181 | 198 |
| 3 | 169 | 203 |
| 4 | 136 | 194 |
| 5 | 169 | 189 |
| 6 | 155 | 197 |
| 7 | 248 | 216 |
| 8 | 168 | 196 |
| 9 | 207 | 212 |
| 10 | 200 | 192 |
| 11 | 159 | 186 |
| 12 | 190 | 199 |
| 13 | 179 | 200 |
| 14 | 140 | 188 |
| 15 | 174 | 188 |
| 16 | 161 | 196 |
| 17 | 256 | 214 |
| 18 | 173 | 188 |
| 19 | 212 | 202 |
| 20 | 201 | 185 |
| Mittelwert der Messungen: | 181,6 | 196,6 |
| Variations-koeffizient: | 17,5% | 4,7% |
| Wiederfindung: | 90,8% | 98% |

Im Mittel werden 98% des eindosierten AFP gefunden. Der Variationskoeffizient ist mit 4,7% sehr niedrig. Die AFP-Konzentrationen werden korrekt wiedergefunden.

Die Nachteile eines AFP-Tests ohne den erfindungsgemäßen Zusatz eines Tensids kommen auch dadurch zum Ausdruck, daß nephelometrische und turbidimetrische Messungen nach dem Stand der Technik zu Ergebnissen führen, die mit denen der vorhandenen AFP-Konzentrationen nicht gut übereinstimmen. Eine nephelometrische Messung für AFP nach dem Stand der Technik unter Verwendung von Latices nach Beispiel 3 im Vergleich mit den eindosierten Konzentrationen an AFP ist mit einem Korrelationskoeffizienten von 0,910 und einer Steigung von 0,83 in der Regressionsanalyse nicht befriedigend.

Werden dagegen die gleiche Zahl nephelometrischer Messungen von AFP unter erfindungsgemäßer Zugabe eines Tensids und zwar von [R]Genapol (1 g/100 g im Testpuffer) durchgeführt, so werden die eindosierten AFP-Mengen in den Patientenseren gut wiedergefunden. Die Steigung in der Regressionsanalyse beträgt 0,99 und der Korrelationskoeffizient 0,998.

Weiterhin wurde Ferritin nephelometrisch nach dem Stand der Technik bestimmt und die Ergebnisse mit denen eines Enzymimmunoassays (Fa. Abbott) verglichen. Die Übereinstimmung zwischen beiden Methoden ist nicht befriedigend. Die Regressionsanalyse ergibt eine Steigung von 0,764 und einen Korrelationskoeffizienten von 0,726. Die mittlere Abweichung für ein Serumkollektiv zwischen beiden Methoden liegt bei 46%.

Wird dagegen ein solcher Ferritin-Test mit 1 g/100 g [R]Genapol im Testpuffer durchgeführt, so findet man eine bessere Übereinstimmung mit den Ergebnissen des Enzymimmunoasssays. Die Regressionsanalyse zeigt eine Steigung von 0,945 und einen Korrelationskoeffizienten von 0,857. Die mittlere Abweichung für ein Serumkollektiv zwischen beiden Methoden liegt bei 20%.

Die folgenden Beispiele erläutern die Erfindung.

Beispiel 1

1. Herstellung von Saatpolymerisat

In ein zylindrisches Glasgefäß, ausgestattet mit Gasein-und Gasauslaßrohr sowie einem Magnetrührstab wurden 310 ml stickstoffgesättigtes bidestilliertes Wasser gegeben. Man gab 500 mg Natriumstearat hinzu und löste dieses durch Rühren. Weiterhin wurden 1,5 ml Ammoniak, 25%ig hinzugegeben. Der pH wurde kontrolliert und betrug 11,09. Durch mehrfaches Evakuieren und Füllen mit Stickstoff wurde das Polymerisationsgefäß Sauerstoff-frei gemacht. Unter ständigem Rühren wurde die Detergenz-Lösung mit Hilfe eines Wasserbades auf +70°C erwärmt. Anschließend gab man 90 ml frisch destilliertes Styrol unter Stickstoff mit Hilfe eines Tropftrichters mit Druckausgleich in das Polymerisationsgefäß. Bei +70°C wurde weitere 15 Minuten zur Emulgation des Styrols gerührt. Anschließend wurde die Temperatur auf +90°C angehoben und eine weitere Stunde gerührt. Danach wurden 67,5 mg Kaliumperoxodisulfat gelöst in 50 ml stickstoffgesättigtem destilliertem Wasser hinzugegeben. Man ließ 130 Minuten bei +90°C rühren. Das Polystyrol wurde durch ein Faltenfilter gegeben. Hierbei bleiben unter Umständen einige ml Styrol auf dem Filter zurück. Dieses Styrol konnte, da etwas im Überschuß vorhanden, nicht vollständig im Polystyrol gelöst werden.

Das filtrierte Polystyrol wurde gegen 10 Liter 0,01%ige Ammoniumhydrogencarbonat-Lösung (mit 0,01% $NH_4HCO_3$; 0,01 Gew.% $NaN_3$; mit 10,5 ml Ammoniak 25 Gew.% in 10 l auf pH 10,0 eingestellt) 50 Stunden lang dialysiert. Man erhielt nach Dialyse 410 ml Polymerisat mit einem Trockengewicht von 17,9 g/dl. Somit sind etwa 85% des eingesetzten Monomeren polymerisiert worden. Durch eine Reduktion der Polymerisationszeit um 5 bis 10 Minuten ließ sich dagegen der Anteil des auspolymerisierten Polystyrols reduzieren, bei gleichzeitiger Erhöhung des im Styrol gelösten Monomeren.

2. Synthese von N-(2,3-Dihydroxypropyl)-Methacrylamid

4,65 g 3-Amino-1,2-propandiol (0,05 mol/l) wurden in 30 ml Dimethylformamid (wasserfrei) gelöst. Diese Lösung sowie 13,8 g $K_2CO_3$ wurden in einem 100 ml Dreihalskolben mit Tropftrichter und Gasein- sowie Gasauslaßrohr gegeben. Die Mischung wurde im Eisbad auf 0°C abgekühlt. Unter mäßigem Rühren und leichtem Durchperlen von Stickstoff wurden während 30 Minuten 6,18 ml Methacrylsäurechlorid (0,06 mol/l), in 30 ml Dimethylformamid gelöst, zugetropft. Nach einer weiteren Stunde Rühren und Eiskühlung ließ man die Mischung sich auf Raumtemperatur erwärmen und rührte weiter 30 Minuten. Der Reaktionsansatz wurde durch ein Faltenfilter filtriert und der Rückstand verworfen. Das Filtrat wurde am Rotationsverdampfer eingeengt, bis ein viskoses Öl entstand. Dieses Öl wurde in 30 ml Methanol gelöst, ein zweites Mal filtriert und das Filtrat wiederum am Rotationsverdampfer eingeengt. Die Reste des Lösungsmittels wurden im Hochvakuum entfernt. Die Ausbeute betrug 8,52 g.

3. Polymerisation von N-(2,3-Dihydroxypropyl)-Methacrylamid (NDPM) auf Saatpolymerisat

In ein zylindrisches Glasgefäß, ausgestattet mit Gasein-und Gasauslaßrohr sowie einem Magnetrührstab, wurden 21,75 ml einer Polystyrol-Latex-Dispersion, hergestellt nach Beispiel 1, mit einem Feststoffgehalt von 18,39 Gew.% sowie 57,25 ml destilliertes Wasser und 50 mg Natriumdodecylsulfat gegeben und durch Rühren gelöst. Durch mehrfaches Evakuieren und Füllen mit Stickstoff wurde das Polymerisationsgefäß Sauerstoff-frei gemacht. Die Latex-Detergenzmischung wurde unter ständigem Rühren auf + 70°C in einem Wasserbad erhitzt. Man gab 1 ml einer Kaliumperoxodisulfatlösung (16 mg/ml in destilliertem Wasser) hinzu.

Es wurde eine Monomerenmischung hergestellt aus: 0,2 ml Styrol, 0,4 ml Methacrylamidoacetaldehyd-di-n-pentylacetal, 0,010 ml Methacrylsäure und 0,4 ml des in 2. erhaltenen N-(2,3-Dihydroxypropyl)-Methacrylamid (NDPM) sowie zur besseren Löslichkeit dieser Monomere 0,2 ml Dimethylformamid.

Unter starkem Rühren der Polystyrol-Latex-Suspension wurde zu dieser die Mischung der Monomere während 60 Minuten langsam hinzugetropft. Die Temperatur des Polymerisationsansatzes wurde auf + 70°C gehalten. Nach dem Zutropfen der Monomerenmischung wurde weitere 5 Stunden bei der genannten Temperatur gerührt. Die Polymerisation war damit beendet und die Dispersion wurde auf Raumtemperatur abgekühlt und mittels Faltenfilter filtriert. Man erhielt 73 ml einer Latex-Suspension. Diese wurde anschließend 17 Stunden lang gegen eine NaHCO$_3$-Pufferlösung (0,25 g/l, pH 8-8,2) dialysiert. Man erhielt 74 ml einer Latex-Dispersion mit einem Feststoffgehalt von 4,7 Gew.%.

4. Polymerisation von 2-Hydroxypropylmethacrylat (HPM) auf Saatpolymerisat

Die Polymerisation erfolgte ähnlich wie in 3. beschrieben. Man stellte eine Mischung von 22,4 ml Polystyrol-Latex hergestellt nach 1., mit einem Feststoffgehalt von 17,9 Gew.% und 56,7 ml destilliertem Wasser sowie 50 mg Natriumdodecylsulfat her. Diese wurde in das Polymerisationsgefäß gegeben und der Sauerstoff entfernt. Weiterhin wurden 1 ml einer Kaliumperoxodisulfatlösung (16 mg/ml in destilliertem Wasser) hinzugegeben und der Ansatz auf + 70°C erhitzt. Man stellte eine Mischung aus 0,4 ml Styrol, 0,4 ml Methacrylamidoacetaldehyd-di-n-pentylacetal, 0,025 ml Methacrylsäure und 0,2 ml 2-Hydroxypropyl-methacrylat (HPM) her. Die Monomerenmischung wurde zur kräftig gerührten Polystyrol-Latex-Suspension bei + 70°C während 60 Minuten langsam hinzugetropft. Anschließend wurde weitere 5 Stunden bei gleicher Temperatur weitergerührt.

Nach Abkühlung auf Raumtemperatur und Filtration durch ein Faltenfilter erhielt man 73 ml des Polymerisats. Man dialysierte anschließend ca. 20 Stunden gegen NaHCO$_3$-Puffer (0,25 g/l, pH 8-8,2). Man erhielt 87 ml einer Latex-Dispersion mit einem Feststoffgehalt von 5,1%.

5. Bindung von Anti-AFP-Antikörpern an ein Polymerisat

An ein Polymerisat unter Verwendung von N-(2,3-Dihydroxypropyl)-methacrylamid hergestellt nach 3. wurden Anti-AFP-Antikörper gebunden.

Das jeweils eingesetzte Polymerisat wurde mit destilliertem Wasser auf einen Feststoffgehalt von 4 Gew.% verdünnt. Ein Antiserum, gewonnen durch Immunisierung von Kaninchen mit aufgereinigtem AFP, wurde nach bekannten Verfahren affinitätschromatographisch aufgereinigt. Es wurde anschließend aufkonzentriert bis ein Proteingehalt von 10 mg/ml erreicht war.

3,4 ml des oben genannten Polymerisates wurden mit 0,34 ml der Anti-AFP-Antikörper-Lösung gemischt. Dann wurden 0,17 ml einer 20%igen wässrigen Lösung von Eikosaoxyethylensorbitan-laurinsäureester ($^R$Tween 20) hinzugegeben und das Ganze nochmals gemischt. Hierzu gab man 0,05 ml 1N HCl, so daß ein pH-Wert von ca. 2 erreicht wurde. Nach einer Inkubationszeit von 30 Minuten bei Raumtemperatur setzte man 0,85 ml gesättigte wässrige Dinatriumhydrogenphosphat-Lösung (pH 6,5) und 0,85 ml wässrige Natriumcyanborhydrid-Lösung (25 mg/ml) hinzu und mischte gut durch. Anschliessend erfolgte eine Inkubation von einer Stunde bei Raumtemperatur.

Dieser Beladungsansatz wurde sodann 30 Minuten mit ca. 50 000 g zentrifugiert (Beckman Zentrifuge, 20 000 UpM). Der Überstand wurde verworfen. Der Rückstand wurde in 5 ml eines Glycin-NaCl-Puffers (0,1 mol/l Glycin, 0,17 mol/l NaCl, 0,5% Eikosaoxyethylen-sorbitan-laurinsäureester ($^R$Tween 20), pH 8,2) resuspendiert. Anschließend erfolgte eine Ultraschall-Behandlung (Bronson Sonyfier B 15) für 2 Sekunden. Das so redispergierte Reagenz wurde im Volumenverhältnis 1:60 mit dem zuvor genannten Glycin-NaCl-Puffer verdünnt.

6. Messung von AFP-Konzentrationen in Serumproben

Das nach 5. durch Bindung von Anti-AFP-Antikörpern an erfindungsgemäßen Latexpräparationen hergestellte Reagenz zur Bestimmung von AFP wurde zur Messung von AFP in aufgestockten Seren eingesetzt. Als Standard wurde das alpha-Fetoprotein Standard-Serum (human) für die Immunpräzipitation mit einer AFP-Konzentration von 322000 ng/ml (Fa. Behringwerke AG) verwendet. Der Standard wurde in einem AFP-freien Serumpool auf 1000 ng/ml verdünnt. Diese Verdünnung wurde in dem AFP-freien Serumpool stufenweise auf jeweils das doppelte Volumen weiter verdünnt. Man erhielt so eine Standardreihe mit abnehmenden AFP-Konzentrationen. Die Standardsera wie auch die zu bestimmenden Patientensera wurden 1:5 in einem Glycin-Kochsalz-Puffer (0,1 mol/l Glycin, 0,17 mol/l NaCl, pH 8,2) verdünnt. Zur Messung wurden 20 $\mu$l Patientenserum-Verdünnung bzw. StandardserumVerdünnung mit 150 $\mu$l eines Reaktionspuffers (0,1 mol/l Glycin, 0,17 mol/l NaCl, 4% Polyethylenglykol (PEG) 6000, 0,5% [R]Tween 20, pH 8,2) in BLN-Küvetten (Fa. Behringwerke AG) pipettiert. Die Küvetten wurden dann nach 12 min Reaktionszeit im Lasernephelometer (Fa. Behringwerke AG) gemessen.

Die Referenzkurve für die Messung des Standardserums wurde auf halblogarithmischem Papier gezeichnet und daran wurden die Meßwerte für die Patientenseren ausgewertet.

Beispiel 2

1. Bindung von Anti-Ferritin-Antikörpern an ein Polymerisat

Ein Polymerisat unter Verwendung von 2-Hydroxypropylmethacrylat hergestellt nach Beispiel 1.4. wurde mit destilliertem Wasser auf einen Feststoffgehalt von 4 Gew.% verdünnt. Ein Antiserum, gewonnen durch Immunisierung von Schafen mit aufgereinigtem Leber-Ferritin, wurde nach bekanntem Verfahren affinitätschromatographisch gereinigt. Es wurde anschließend aufkonzentriert, bis ein Proteingehalt von 12 mg/ml erreicht war.

0,5 ml des oben genannten Polymerisats wurden mit 0,050 ml der Anti-Leber-Ferritin-Antikörper-Lösung (verdünnt auf 5 mg/ml mit isotonischer Kochsalz-Lösung) gemischt. Dann wurden 0,025 ml einer 20%igen wässrigen Lösung von Eikosaoxyethylen-sorbitan-laurinsäureester ([R]Tween 20) hinzugegeben und das Ganze nochmals gemischt. Hierzu gaben wir 0,010 ml 1 N HCl, so daß ein pH-Wert von ca. 2 erreicht wurde. Nach einer Inkubationszeit von 30 Minuten bei Raumtemperatur setzten wir 0,125 ml gesättigte wässrige Dinatriumhydrogenphosphat-Lösung (pH 6,5) und 0,125 ml wässrige Natriumcyanborhydrid-Lösung (25 mg/ml) hinzu und mischten gut durch. Anschließend erfolgte eine Inkubation von einer Stunde bei Raumtemperatur. Dieser Beladungsansatz wurde sodann 30 Minuten mit ca. 50 000 g zentrifugiert (Beckman Zentrifuge, 20 000 UpM). Der Überstand wurde verworfen. Der Rückstand wurde in 0,75 ml eines Glycin-NaCl-Puffers (0,1 mol/l Glycin, 0,17 mol/l NaCl, 0,5% Eikosaoxyethylen-sorbitan-laurinsäureester ([R]Tween 20) pH 8,2) resuspendiert.

Anschließend erfolgte eine Ultraschall-Behandlung (Bronson Sonyfier B 15) für 2 Sekunden. Das so redispergierte Reagenz wurde im Volumenverhältnis 1:30 mit 0,05 mol/l Imidazol + 6% Saccharose + 0,1% Human-Albumin vorverdünnt und weiter 1:2 mit Aqua dest. verdünnt.

2. Messung von Ferritin-Konzentrationen in Serumproben

Das durch Bindung von Anti-Leber-Ferritin-Antikörpern an die Latexpräparation hergestellte Reagenz zur Bestimmung von Ferritin wurde zur Messung von Ferritin in Patientenseren eingesetzt.

Als Standard wurde eine Ferritin-Standardabfüllung des Enzygnost Tests (Fa. Behringwerke AG) verwendet. Dieser Ferritin-Standard enthielt 20 000 ng/ml. Der Standard wurde in einem Ferritin-freien Serumpool zunächst auf 10 000 ng/ml und dann weiter auf 1250 ng/ml verdünnt. Von diesem Punkt wurde eine geometrische Verdünnungsreihe (Faktor 2, 5 Verdünnungsstufen) angelegt. Die Standardseren wie auch die zu bestimmenden Patientenseren wurden 1:5 in einem Glycin-Kochsalzpuffer (0,1 mol/l Glycin, 0,17 mol/l NaCl, pH 8,2) verdünnt. Zur Messung wurden 20 $\mu$l Patientenserum-Verdünnung bzw. Standardserum-Verdünnung mit 150 $\mu$l eines Reaktionspuffers (0,17 mol/l NaCl, 0,1 mol/l Glycin, 0,11% Na-Azid, + 0,2% RSA + 0,02% Benzamidiniumchlorid 4% PEG 6000, 0,5% [R]Tween 20, pH 8,2) mit Zusatz von 1% [R]Genapol T 250 (Fa. Hoechst AG) in BLN-Küvetten (Fa. Behringwerke AG) gemischt und 30 Minuten bei Raumtemperatur inkubiert. Die Küvetten wurden dann im Lasernephelometer (Fa. Behringwerke AG) gemessen.

Die Referenzkurve für die Messung der Standardseren wurde auf halblogarithmischem Papier gezeichnet und daran wurden die Meßwerte für die Patientenseren ausgewertet.

**Patentansprüche**

1. Verwendung einer Verbindung der Formel I

$CH_3$-$(CH_2)_m$-R-$(CH_2$-$CH_2$-O$)_n$-H      I

worin
  R = NH oder CH=CH-$(CH_2)_p$-O mit
  m = 3 - 26,
  n = 7 - 40 und
  p = 5 - 15 sind,
in einer Konzentration von mehr als 0,01 g/100 g in einem Verfahren zur Bestimmung eines Partners einer immunchemischen Reaktion, in welchem einer der Partner in einer wässrigen Flüssigkeit gelöst mit dem anderen Partner, der kovalent an eine Festphase gebunden ist, zur Reaktion gebracht wird zur Vermeidung von Störungen der Bestimmung, die durch Adsorption unspezifischer Proteine an die Festphase auftreten.

2. Verwendung nach Anspruch 1, dadurch gekennzeichnet, daß
  R = CH=CH-$(CH_2)_p$-O und
  m = 3-26, n = 7-40 und p = 5-15 sind.

3. Verwendung nach Anspruch 1, dadurch gekennzeichnet, daß
  R = CH=CH-$(CH_2)_p$-O und
  m = 15-17, n = 25 und p = 6-8 sind.

4. Verwendung nach Anspruch 1, dadurch gekennzeichnet, daß
  R = CH=CH-$(CH_2)_p$-O und
  m = 6-8, n = 25 und p = 6-8 sind.

5. Verwendung nach Anspruch 1, dadurch gekennzeichnet, daß eine handelsübliche Mischung ([R]Genapol T250) von Verbindungen der Formel I, die 20-35 % Palmitylalkohol veräthert mit Polyethylenglycol des Polymerisationsgrads P = 25, 15-30 % Stearylalkohol veräthert mit Polyethylenglycol (P = 25) und 30-60 % Oleylalkohol veräthert mit Polyethylenglycol (P = 25) enthält, verwendet wird.

6. Verwendung nach Anspruch 1, dadurch gekennzeichnet, daß während der Reaktion Glycin in einer Konzentration von 0,05-0,2 mol/l, ein Neutralsalz in einer Konzentration von 0,05-0,5 mol/l, Polyethylenglycol in einer Konzentration von 4 g/100 g und/oder Eikosa-sorbitan-laurylsäureester ([R]Tween 20)in einer Konzentration von 0,2-1 g/100 g vorhanden sind.

**Claims**

1. The use of a compound of the formula I

$CH_3$-$(CH_2)_m$-R-$(CH_2$-$CH_2$-O$)_n$-H      I

in which R is NH or CH=CH-$(CH_2)_p$-O with m being 3 - 26, n being 7 - 40 and p being 5 - 15, in a concentration of more than 0.01 g/100 g, in a method for the determination of a partner in an immunochemical reaction, in which one of the partners, which is dissolved in an aqueous fluid, is allowed to react with the other partner, which is covalently bonded to a solid phase, in order to avoid interference with the determination occurring due to adsorption of nonspecific proteins onto the solid phase.

2. The use as claimed in claim 1, wherein R is CH=CH-$(CH_2)_p$-O and m is 3-26, n is 7-40 and p is 5-15.

3. The use as claimed in claim 1, wherein R is CH=CH-$(CH_2)_p$-O and m is 15-17, n is 25 and p is 6-8.

4. The use as claimed in claim 1, wherein R is CH=CH-$(CH_2)_p$-O and m is 6-8, n is 25 and p is 6-8.

5. The use as claimed in claim 1, in which is used a commercially available mixture ([R]Genapol T250) of compounds of the formula I which contains 20-35 % of palmityl alcohol etherified with polyethylene glycol with a degree of polymerization of p = 25, 15-30 % of stearyl alcohol etherified with polyethylene glycol (P = 25) and 30-60 % of oleyl alcohol etherified with polyethylene glycol (P = 25).

6. The use as claimed in claim 1, in which there are present during the reaction glycine in a concentration of 0.05-0.2 mol/l, a neutral salt in a concentration of 0.05-0.5 mol/l, polyethylene glycol in a concentration of 4 g/100 g and/or eicosaoxyethylene sorbitan laurate ([R]Tween 20) in a concentration of 0.2-1 g/100 g.

**Revendications**

1. Utilisation d'un composé de formule I

$$CH_3\text{-}(CH_2)_m\text{-}R\text{-}(CH_2\text{-}CH_2\text{-}O)_n\text{-}H \qquad I$$

dans laquelle
$R = NH$ ou $CH=CH\text{-}(CH_2)_p\text{-}O$ avec $m = 3\text{-}26$,
$n = 7\text{-}40$ et
$p = 5\text{-}15$,
à une concentration de plus de 0,01 g/100 g, dans un procédé pour la détermination d'un partenaire d'une réaction immunochimique, dans lequel on met en réaction l'un des partenaires, dissous dans un liquide aqueux, avec l'autre partenaire, qui est lié par covalence à une phase solide, pour éviter des perturbations de la détermination qui apparaissent par adsorption de protéines non spécifiques sur la phase solide.

2. Utilisation selon la revendication 1, caractérisée en ce que $R = CH=CH\text{-}(CH_2)_p\text{-}O$ et $m = 3\text{-}26$, $n = 7\text{-}40$ et $p = 5\text{-}15$.

3. Utilisation selon la revendication 1, caractérisée en ce que $R = CH=CH\text{-}(CH_2)_p\text{-}O$ et $m = 15\text{-}17$, $n = 25$ et $p = 6\text{-}8$.

4. Utilisation selon la revendication 1, caractérisée en ce que $R = CH=CH\text{-}(CH_2)_p\text{-}O$ et $m = 6\text{-}8$, $n = 25$ et $p = 6\text{-}8$.

5. Utilisation selon la revendication 1, caractérisée en ce que l'on utilise un mélange du commerce (Genapol® T 250) de composés de formule I, qui contient 20-35 % d'alcool palmitylique éthérifié avec un polyéthylèneglycol ayant un degré de polymérisation P = 25, 15-30 % d'alcool stéarylique éthérifié avec un polyéthylèneglycol (P = 25) et 30-60 % d'alcool oléylique éthérifié avec un polyéthylèneglycol (P = 25).

6. Utilisation selon la revendication 1, caractérisée en ce que, pendant la réaction sont présents de la glycine à une concentration de 0,05 - 0,2 mole/l, un sel neutre à une concentration de 0,05 - 0,5 mole/l, du polyéthylèneglycol à une concentration de 4 g/100 g et/ou du monolaurate polyoxyéthylénique(20) de sorbitanne (Tween® 20) à une concentration de 0,2 - 1 g/100 g.